**Europäisches Patentamt**

**European Patent Office**

**Office européen des brevets**

(19)

(11) Veröffentlichungsnummer: **0 037 478**
**B1**

(12)

# EUROPÄISCHE PATENTSCHRIFT

(45) Veröffentlichungstag der Patentschrift :
15.12.82

(51) Int. Cl.³ : **A 23 K 1/18, A 61 K 9/42**

(21) Anmeldenummer : **81101880.3**

(22) Anmeldetag : **13.03.81**

(54) **Pansendurchtretender Futterzusatz für Wiederkäuer und dessen Verwendung zur Fütterung von Wiederkäuern.**

(30) Priorität : **03.04.80 DE 3013000**

(43) Veröffentlichungstag der Anmeldung :
**14.10.81 (Patentblatt 81/41)**

(45) Bekanntmachung des Hinweises auf die Patenterteilung : **15.12.82 Patentblatt 82/50**

(84) Benannte Vertragsstaaten :
**AT BE CH FR GB IT LI NL SE**

(56) Entgegenhaltungen :
**DE A 1 692 452**
**DE B 2 212 568**
**DE C 287 292**
**FR A 948 590**
**US A 2 373 763**
**US A 3 037 911**
**US A 3 655 864**

(73) Patentinhaber : **Degussa Aktiengesellschaft**
**Weissfrauenstrasse 9**
**D-6000 Frankfurt am Main 1 (DE)**

(72) Erfinder : **Spindler, Manfred, Dr.Dipl.-Chem.**
**Kurfürstenstrasse 24**
**D-6450 Hanau 1 (DE)**
Erfinder : **Tanner, Herbert, Dr.Dipl.-Chem.**
**Wildaustrasse 20**
**D-6450 Hanau 9 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Pansendurchtretender Futterzusatz für Wiederkäuer und dessen Verwendung zur Fütterung von Wiederkäuern

Gegenstand der Erfindung ist ein pansendurchtretender Futterzusatz für Wiederkäuer in Teilchenform, bei dem mindestens eine biologisch wirksame Substanz mit einem Überzug versehen ist, der ein Salz einer aliphatischen Monocarbonsäure mit 14 bis 22 Kohlenstoffatomen oder der Ricinolsäure oder eines Gemisches mehrerer der genannten Säuren enthält, sowie die Verwendung dieses Futterzusatzes zur Fütterung von Wiederkäuern.

Aus der DE-C22 12 568 ist bereits ein pansendurchtretender Futterzusatz für Wiederkäuer in Teilchenform bekannt, bei dem mindestens eine biologisch wirksame Substanz mit einem Überzug versehen ist, welcher den Umgebungsbedingungen des Pansens gegenüber widerstandsfähig ist, aber nach dem Durchtritt in den Darm unter Einwirkung von Galle und Pankreas-Saft aufgelöst wird. Der Überzug besteht aus einer aliphatischen Monocarbonsäure mit 14 bis 22 Kohlenstoffatomen oder Ricinolsäure oder einem Gemisch solcher Säuren oder aus einem Salz einer solchen Säure oder eines Gemisches solcher Säuren. Dadurch, dass die Auflösung des Überzuges bei dem bekannten Futterzusatz die Einwirkung von Galle und Pankreas-Saft erfordert, wird jedoch die für die Verdauung und die Resorption zur Verfügung stehende Zeit beträchtlich verkürzt.

Der erfindungsgemässe Futterzusatz ist nun dadurch gekennzeichnet, dass er besteht aus, jeweils bezogen auf das Gesamtgewicht,

a) 30 bis 50 Gewichtsprozent mindestens einer biologisch wirksamen Substanz,

b) 10 bis 35 Gewichtsprozent mindestens eines Natrium-, Kalium- oder Calciumsalzes einer aliphatischen Monocarbonsäure mit 14 bis 22 Kohlenstoffatomen oder der Ricinolsäure und

c) Rest bis auf 100 Gewichtsprozent, mindestens aber 30 Gewichtsprozent mindestens einer aliphatischen Monocarbonsäure mit 14 bis 22 Kohlenstoffatomen und/oder Ricinolsäure und/oder eines gehärteten pflanzlichen oder tierischen Fettes.

Es hat sich überraschend gezeigt, dass die Teilchen des erfindungsgemässen Futterzusatzes im Gegensatz zu solchen, bei denen der Überzug entweder nur aus Fettsäuren oder nur aus Salzen von Fettsäuren besteht, schon im sauren Milieu des Labmagens angegriffen werden und die biologisch wirksamen Substanzen freisetzen, ohne dass es der zusätzlichen Einwirkung von Galle und Pankreas-Saft bedarf. Andererseits aber widerstehen auch die Teilchen des erfindungsgemässen Futterzusatzes einem Angriff durch ein Milieu mit dem pH des Pansensaftes und dem mikrobiellen Abbau im Pansen. Wie die nachfolgenden Beispiele belegen, liegt bei Anwendung gleicher, in den Beispielen beschriebener Testbedingungen der im Pansenmilieu ungelöste Anteil bei dem erfindungsgemässen Futterzusatz im allgemeinen etwas höher als bei dem jeweiligen Vergleichsversuch. Die Teilchen des erfindungsgemässen Futterzusatzes können daher ebenso wie die aus der DE-C-22 12 568 bekannten Teilchen durch den Pansen hindurchtreten, beginnen sich jedoch schon im Labmagen aufzulösen, wodurch die für die Verdauung und Resorption zur Verfügung stehende Zeit verlängert wird.

Der erfindungsgemässe Futterzusatz besteht aus, bezogen auf das Gesamtgewicht, 30 bis 50 Gewichtsprozent mindestens einer biologisch wirksamen Substanz. Solche biologisch wirksame Substanzen sind beispielsweise Aminosäuren, wie Methionin oder Lysin ; Derivate von Aminosäuren, wie N-Acylaminosäuren, zum Beispiel N-Stearoylmethionin oder N-Oleoylmethionin ; das Calciumsalz des N-Hydroxymethylmethionins oder Lysin-monohydrochlorid ; hydroxyanaloge Verbindungen von Aminosäuren, wie 2-Hydroxy-4-methylmercaptobuttersäure oder deren Calciumsalz ; Proteine, wie Federmehl, Fischmehl, Casein oder Kartoffeleiweiß ; Vitamine, wie Vitamin A, Vitamin A-Acetat, Vitamin A-Palmitat, Vitamin $D_3$, Vitamin E, Nicotinsäure oder Nicotinsäureamid, Calciumpanthotenat ; B-Carotin ; Enzyme, wie saure Proteasen ; Kohlehydrate, wie Glucose ; Veterinärpharmazeutika, zum Beispiel Antibiotika, wie Chlortetracyclin, Oxytetracyclin, Chloramphenicol, Zinkbacitracin ; Anthelmintika, wie Piperazinsalze ; Antiparasitika, wie Neguvon [(2,2,2-Trichlor-1-hydroxy-äthyl)-phosphorsäuredimethylester]. Es können selbstverständlich auch Gemische aus zwei oder mehr biologisch wirksamen Substanzen angewandt werden.

Der die biologisch wirksamen Substanzen im Pansen schützende Überzug enthält, ebenfalls bezogen auf das Gesamtgewicht des Futterzusatzes, 10 bis 35 Gewichtsprozent mindestens eines Natrium-, Kalium- oder Calciumsalzes einer aliphatischen Monocarbonsäure mit 14 bis 22 Kohlenstoffatomen oder der Ricinolsäure und zur Ergänzung auf 100 Gewichtsprozent, mindestens aber, bezogen auf das Gesamtgewicht des Futterzusatzes, 30 Gewichtsprozent mindestens einer aliphatischen Monocarbonsäure mit 14 bis 22 Kohlenstoffatomen und/oder Ricinolsäure und/oder eines gehärteten pflanzlichen oder tierischen Fettes, zum Beispiel gehärtetes Sojabohnenöl oder gehärteten Rindertalg.

Die im schützenden Überzug als freie Fettsäure oder in Form der Natrium-, Kalium- oder Calciumsalze vorliegenden aliphatischen Monocarbonsäuren mit 14 bis 22 Kohlenstoffatomen können gesättigt oder ungesättigt, verzweigt oder unverzweigt sein. Bevorzugt werden die unverzweigten, in natürlichen Fetten vorkommenden Fettsäuren, wie Myristinsäure, Palmitinsäure, Stearinsäure, Arachinsäure oder Behensäure, oder Gemische solcher Säuren.

Der erfindungsgemässe Futterzusatz kann zweckmässigerweise so hergestellt werden, dass zunächst eine homogene Schmelze aus den den schützenden Überzug bildenden Komponenten bereitet wird. In dieser Schmelze wird dann die zu schützende biologisch wirksame Substanz bzw. ein Gemisch solcher Substanzen unter Rühren dispergiert. Anschliessend wird die flüssige Dispersion, die durch Vibrationen hoher Frequenz in Schwingung versetzt wird, aus Düsen ausfliessen lassen. Dabei wird der aus jeder düse austretende Strahl in diskrete Tröpfchen definierter Grösse aufgeteilt, die nach Ausbildung einer Kugelform im freien Fall erstarren. Auf diese Weise lassen sich kugelförmige Teilchen mit einem beliebigen Durchmesser zwischen 50 und 2 500 $\mu$m in einem engen Kornspektrum herstellen.

Eine in Form des erfindungsgemässen Futterzusatzes besonders wichtige biologisch wirksame Substanz ist das Methionin. Denn zahlreiche Untersuchungen belegen, dass von allen Aminosäuren in der Regel Methionin die beim Widerkäuer erstlimitierende Aminosäure ist. So führt die Infusion von Methionin in den Labmagen von Schafen zu einer Steigerung des Wollwachstums [P.J. Reis, P.G. Schinckel, Aust. J. Biol. Sci. *16*, 218 (1963)]. Bei Holstein-Ochsen führt die Infusion von Methionin in den Labmagen zu einer erhöhten N-Bilanz [C.R. Richardson, E.E. Hatfield, J. Anim. Sci. *46*, 740 (1978)].

Untersuchungen an laktierenden Kühen sind bei W. Kaufmann und W. Lüpping in « Z. Tierphysiol., Tierernährg. u. Futtermittelkde. » 41, 202 (1979) zitiert. Dort sind auch Ergebnisse von Versuchen angegeben, welche belegen, dass ein während des Durchtritts durch den Pansen geschütztes Methionin, aus dem jedoch im Labmagen das Methionin freigesetzt wird, als Zusatz zu einer herkömmlichen Kraftfutterration bei Milchkühen eine Steigerung der Milchleistung, des Protein- und Fettgehaltes der Milch, der Persistanz und der Fruchtbarkeit nach Beginn der Lactation bewirkt.

Ein weiterer Gegenstand der Erfindung ist daher die Verwendung des erfindungsgemässen Futterzusatzes, vorzugsweise mit Methionin als biologisch wirksamer Substanz, in einer täglichen Dosis von 1 bis 100 g zur Fütterung von Wiederkäuern, insbesondere von Milchkühen zur Verbesserung der Milchleistung, des Protein- und Fettgehaltes der Milch, der Persistenz und/oder der Fruchtbarkeit nach Beginn der Lactation.

Weitere wichtige biologisch wirksame Verbindungen sind die Vitamine. So führt zum Beispiel Mangel an Vitamin A bei Rindern zu schlechter Freßlust und deshalb geringen Gewichtszunahmen. Bei trächtigen Kühen kann er Verkalben oder totgeborene Kälber bewirken. $\beta$-Carotin hat eine spezifische Wirkung auf die Fruchtbarkeit von Rindern [Dt. Tierärztl. Wschr. *82*, 444 (1975)].

Eine energetische Unterversorgung hochleistender Tiere kann zu verringerter Milcheiweißleistung führen. Zufuhr von Kohlehydraten, wie Glucose, in geschützter Form führt hierbei zu einer besonders günstigen Energieversorgung.

Bei oral verabreichten Medikamenten ist eine Freisetzung der Wirksubstanz in der Regel erst nach Passage des Pansens erwünscht. Der erfindungsgemäße Futterzusatz ist insbesondere dann geeignet, wenn eine Freisetzung im Labmagen erwünscht ist.

Die Erfindung soll durch die nachfolgenden Beispiele und Vergleichsversuche näher verdeutlicht werden. Soweit nicht anders angegeben, bedeuten Teile Gewichtsteile und Prozente Gewichtsprozente.

Beispiel 1

a) (Vergleichsversuch) 70 Teile Stearinsäure wurden bei 80 °C geschmolzen. 30 Teile Methionin wurden unter Rühren einsuspendiert und die Schmelze aus Düsen getropft, wobei gleichmässige kugelförmige Teilchen von 1 bis 2 mm Durchmesser entstanden.

b) Aus einer Mischung von 60 Teilen Stearinsäure und 10 Teilen Natriumstearat wurde durch Erhitzen auf 80 °C eine homogene Schmelze hergestellt, in die ebenfalls 30 Teile Methionin eingerührt wurden. Die Herstellung der Teilchen erfolgte wie nach a).

In analoger Weise wurden Teilchen hergestellt aus :

c) 30 Teilen Methionin, 55 Teilen Stearinsäure und 15 Teilen Natriumstearat,
d) 30 Teilen Methionin, 50 Teilen Stearinsäure und 20 Teilen Natriumstearat,
e) 30 Teilen Methionin, 50 Teilen Stearinsäure und 20 Teilen Kaliumstearat,
f) 30 Teilen Methionin, 45 Teilen Stearinsäure und 25 Teilen Natriumstearat,
g) 30 Teilen Methionin, 35 Teilen Stearinsäure und 35 Teilen Natriumstearat.

Zur Untersuchung der spezifisch pH-abhängigen Freisetzung durch Ausnutzung des pH-Unterschiedes zwischen Pansensaft und Labmagensaft wurden die Teilchen jeweils 2 Stunden bei 37 °C in 50 ml eines Puffersystems, das pH-mässig dem Pansenmilieu entspricht, unter ständiger Bewegung in einer Schüttelapparatur inkubiert. Das Puffersystem bestand aus einer Lösung von 74,77 g Kaliumdihydrogenphosphat, 103,03 g Dinatriumhydrogenphosphattrihydrat und 13,5 g Kaliumjodid in 1 l Wasser. Der pH-Wert des Puffersystems betrug 6,5. Gleichzeitig wurden die Teilchen 2 Stunden bei 37 °C in 50 ml eines Puffersystems inkubiert, das pH-mässig dem Labmagenmilieu entspricht. Dieses System bestand aus einer Lösung von 6,5 ml 0,2 N Salzsäure und 25,0 ml 0,2 N Kaliumchloridlösung in 100 ml Wasser. Der pH-Wert dieses Puffersystems betrug 2,0.

Anschliessend wurden die nicht aufgelösten Teilchen abfiltriert und die Lösungen mit jeweils 100 ml Phosphatpuffer vom pH 6,5 versetzt, wobei jeweils ein pH-Wert von 6,5 resultierte. Der Anteil des aus den Teilchen in Lösung gegangenen Methionins wurde anschliessend jodometrisch bestimmt.

Die Ergebnisse sind in folgender Tabelle 1 aufgeführt :

TABELLE 1

| Versuch | Zusammensetzung des Produktes | | | Ungelöster Anteil des Methionins in % | |
|---|---|---|---|---|---|
| | Methionin | Stearinsäure | Stearat | Pansenpuffer | Labmagenpuffer |
| 1 a)* | 30 | 70 | — | 79.8 | 96.7 |
| 1 b) | 30 | 60 | 10 (Na) | 83.4 | 94.9 |
| 1 c) | 30 | 55 | 15 (Na) | 85.5 | 80.9 |
| 1 d) | 30 | 50 | 20 (Na) | 86.1 | 16.3 |
| 1 e) | 30 | 50 | 20 (K) | 85.7 | 24.5 |
| 1 f) | 30 | 45 | 25 (Na) | 88.2 | 64.8 |
| 1 g) | 30 | 35 | 35 (Na) | 87.5 | 49.6 |

\* Vergleichsversuch

Das Produkt mit einem Anteil von 20 % Stearat zeigt eine sehr weitgehende Freisetzung des Methionins durch die pH-Verhältnisse im Labmagen, ohne dass enzymatische Aktivität notwendig wäre. Ein niedrigerer oder höherer Anteil an Stearat führt zu einer weniger effektiven Freisetzung des Methionins während der Inkubationszeit von nur zwei Stunden.

Dieser Effekt ist auch optisch sichtbar : Während die Teilchen nach a) nach Inkubation im sauren Puffer keine äussere Veränderung zeigten, waren die Teilchen nach d) und e) an vielen Stellen aufgeplatzt. Die Teilchen nach b), f) und g) zeigten eine geringe Aufweichung der äusseren Schicht.

Beispiel 2

In analoger Weise wurden Teilchen mit einem Methionin-Anteil von 40 %, bezogen auf das Endprodukt, hergestellt. Der Anteil an Natriumstearat wurde auf 10, 15 und 20 % eingestellt. Zum Vergleich wurde ein Produkt ohne Stearatzusatz hergestellt und untersucht.

Die Zusammensetzung und die Ergebnisse sind in der Tabelle 2 aufgeführt.

TABELLE 2

| Versuch | Zusammensetzung des Produktes | | | Ungelöster Anteil des Methionins in % | |
|---|---|---|---|---|---|
| | Methionin | Stearinsäure | Stearat | Pansenpuffer | Labmagenpuffer |
| 2 a)* | 40 | 60 | — | 71.7 | 95.1 |
| 2 b) | 40 | 50 | 10 (Na) | 84.6 | 92.3 |
| 2 c) | 40 | 45 | 15 (Na) | 85.2 | 49.1 |
| 2 d) | 40 | 40 | 20 (Na) | 88.1 | 39.9 |

\* Vergleichsversuch

Ein höherer Anteil an Stearat als in den Beispielen beschrieben, führt zu einer Erhöhung der Viskosität der Schmelze, so dass die Herstellung auch grösserer Teilchen erschwert wird. Bei einem vollständigen Ersatz der Fettsäure durch ein Salz der Fettsäure ist auch bei Anwendung von Temperaturen von 180 °C eine Schmelze nicht herstellbar. Dies führt darüberhinaus zu einer wesentlich grösseren thermischen Belastung der zu schützenden Substanz.

Beispiel 3

Zum Vergleich des oben beschriebenen Tests mit dem Ergebnis bei Anwendung physiologischer Lösungen wird das Produkt nach Versuch 1 d) mit 50 ml Labmagensaft, erhalten von frisch geschlachteten Rindern, ebenfalls bei 37 °C 2 Stunden inkubiert. Die Bestimmung erfolgt in gleicher Weise. Das Ergebnis zeigt Tabelle 3 :

TABELLE 3

| Versuch | Zusammensetzung des Produktes | | | Ungelöster Anteil des Methionins in % | |
|---|---|---|---|---|---|
| | Methionin | Stearinsäure | Stearat | Labmagenpuffer | Labmagensaft |
| 3 a) und b) | 30 | 50 | 20 (Na) | 16.3 | 26.2 |

## Beispiel 4

Zur Beurteilung, ob bei dem nach Versuch 1 b) erhaltenen Produkt mit einem Anteil von 10 % Natriumstearat nach längerer Inkubation im Labmagensaft auch eine gegenüber dem Produkt nach Versuch 1 a) (ohne Stearatanteil) erhöhte Freisetzung beobachtet werden kann, wurden beide Produkte jeweils 16 Stunden bei 37 °C in Labmagensaft inkubiert. Dabei ergab sich folgendes Ergebnis (Tabelle 4) :

### TABELLE 4

| Versuch | Zusammensetzung des Produktes | | | Ungelöster Anteil des Methionins in % (Labmagensaft, 16 h) |
|---|---|---|---|---|
| | Methionin | Stearinsäure | Stearat | |
| 4 a)* | 30 | 70 | — | 72.7 |
| 4 b) | 30 | 60 | 10 (Na) | 25.3 |

* Vergleichsversuch

## Beispiel 5

In analoger Weise wie bei Beispiel 1 und 2 wurde ein Produkt, bestehend a) aus 30 % Lysinhydrochlorid und 70 % Stearinsäure, sowie b) aus 30 % Lysinhydrochlorid, 50 % Stearinsäure und 20 % Natriumstearat, hergestellt.

Nach Inkubation in Labmagenpuffer, 2 Stunden bei 37 °C, wurden die Teilchen abfiltriert und die Lösungen auf dem Aminosäureanalysator LKB 3201 säulenchromatographisch auf den Gehalt an Lysin untersucht.

Dabei ergaben sich folgende Unterschiede in der Freisetzungsrate (Tabelle 5) :

### TABELLE 5

| Versuch | Zusammensetzung des Produktes | | | Ungelöster Anteil des Lysinhydrochlorids in % (Labmagenpuffer, 2 h) |
|---|---|---|---|---|
| | Lys · HCl | Stearinsäure | Stearat | |
| 5 a)* | 30 | 70 | — | 77.1 |
| 5 b) | 30 | 70 | 20 (Na) | 22.0 |

* Vergleichsversuch

## Beispiel 6

Entsprechend Beispiel 1 wurden Teilchen hergestellt
a) aus 30 % Nicotinsäureamid und 70 % Stearinsäure sowie aus b) 30 % Nicotinsäureamid, 50 % Stearinsäure und 20 % Natriumstearat.

Nach Inkubation in Labmagensaft 2 Stunden bei 37 °C, wurden die Teilchen abfiltriert und der N-Gehalt mit der Kjeldahl-Methode bestimmt. Durch Vergleich mit dem vor der Inkubation analysierten N-Gehalt wurde das Ausmass der Freisetzung festgestellt. Das Ergebnis zeigt Tabelle 6 :

### TABELLE 6

| Versuch | Zusammensetzung des Produktes | | | Ungelöster Anteil des Nicotinsäureamids in % (Labmagenpuffer, 2 h) |
|---|---|---|---|---|
| | Nicotinsäureamid | Stearinsäure | Stearat | |
| 6 a)* | 30 | 70 | — | 94.1 |
| 6 b) | 30 | 50 | 20 (Na) | 9.3 |

* Vergleichsversuch

## Beispiel 7

In analoger Weise wie bei Beispiel 1 und 2 wurden Teilchen hergestellt, bei denen in der schützenden Schicht gehärtetes Sojaöl verwendet wurde.

Die Zusammensetzung und das Ergebnis des wie in Beispiel 1 durchgeführten Tests zeigt Tabelle 7 :

**0 037 478**

TABELLE 7

| Versuch | Zusammensetzung des Produktes | | | Ungelöster Anteil des Methionins in % | |
| | Methionin | gehärtetes Sojaöl | Stearat | Pansenpuffer | Labmagenpuffer |
|---|---|---|---|---|---|
| 7 a)* | 30 | 70 | — | 96.7 | 94.6 |
| 7 b) | 30 | 60 | 10 (K) | 94.0 | 57.9 |
| 7 c) | 30 | 50 | 20 (K) | 69.0 | 49.7 |

* Vergleichsversuch

## Beispiel 8

In analoger Weise, wie oben beschrieben, wurden Produkte hergestellt, bei denen Methionin und Protein in Kombination geschützt wurden. Als Protein wurde Federmehl verwendet.

Nach Durchführung der Inkubation in Pansen- und Labmagenpuffer wie bei Beispiel 1 wurden die Lösungen säulenchromatographisch auf den Aminosäurengehalt untersucht. Neben Methionin waren andere Aminosäuren nur in Spuren nachweisbar.

Die Zusammensetzung und das Ergebnis zeigt Tabelle 8 :

TABELLE 8

| Versuch | Zusammensetzung des Produktes | | | | Ungelöster Anteil des Methionins in % | |
| | Methionin | Federmehl | Stearinsäure | Stearat | Pansenpuffer | Labmagenpuffer |
|---|---|---|---|---|---|---|
| 8 a)* | 30 | 20 | 50 | — | 60.7 | 75.9 |
| 8 b) | 30 | 20 | 40 | 10 (Na) | 69.4 | 8.2 |
| 8 c) | 30 | 20 | 30 | 20 (Na) | 74.6 | 8.7 |

* Vergleichsversuch

## Ansprüche

1. Pansendurchtretender Futterzusatz für Wiederkäuer in Teilchenform, bei dem mindestens eine biologisch wirksame Substanz mit einem Überzug versehen ist, der ein Salz einer aliphatischen Monocarbonsäure mit 14 bis 22 Kohlenstoffatomen oder der Ricinolsäure oder eines Gemisches mehrerer der genannten Säuren enthält, dadurch gekennzeichnet, dass er besteht aus, jeweils bezogen auf das Gesamtgewicht,

a) 30 bis 50 Gewichtsprozent mindestens einer biologisch wirksamen Substanz,

b) 10 bis 35 Gewichtsprozent mindestens eines Natrium-, Kalium- oder Calciumsalzes einer aliphatischen Monocarbonsäure mit 14 bis 22 Kohlenstoffatomen oder der Ricinolsäure und

c) Rest bis auf 100 Gewichtsprozent, mindestens aber 30 Gewichtsprozent mindestens einer aliphatischen Monocarbonsäure mit 14 bis 22 Kohlenstoffatomen und/oder Ricinolsäure und/oder eines gehärteten pflanzlichen oder tierischen Fettes.

2. Verwendung des Futterzusatzes nach Anspruch 1 in einer täglichen Dosis von 1 bis 100 g zur Fütterung von Wiederkäuern, insbesondere von Milchkühen zur Verbesserung der Milchleistung, des Protein- und Fettgehaltes der Milch, der Persistenz und/oder der Fruchtbarkeit nach Beginn der Lactation.

## Claims

1. A particulate rumen-penetrating feed additive for ruminants in which at least one biologically active substance is provided with a coating containing a salt of an aliphatic monocarboxylic acid containing from 14 to 22 carbon atoms or of ricinoleic acid or of a mixture of several such acids, characterised in that it contains — based in each case of the total weight —

a) from 30 to 50 % by weight of at least one biologically active substance,

b) from 10 to 35 % by weight of at least one sodium, potassium or calcium salt of an aliphatic $C_{14}$-$C_{22}$ monocarboxylic acid or of ricinoleic acid and

6

c) the balance to 100 % by weight, but at least 30 % by weight of at least one aliphatic $C_{14}$-$C_{22}$ monocarboxylic acid or ricinoleic acid and/or of a hardened vegetable or animal fat.

2. The use of the feed additive claimed in Claim 1 in a daily dose of from 1 to 100 g for feeding ruminants, particularly milk cows for improving milk output, the protein and fat content of the milk, persistence and/or fecundity after the beginning of lactation.

**Revendications**

1. Complément alimentaire traversant la panse pour ruminants, sous forme particulaire, dans lequel au moins une substance biologiquement active est garnie d'un enrobage, qui contient un sel d'un acide carboxylique aliphatique avec 14 à 22 atomes de carbone ou de l'acide ricinoléique ou d'un mélange de plusieurs des acides sus-mentionnés, caractérisé en ce qu'il se compose, chaque fois rapporté au poids total,

a) de 30 à 50 % en poids d'au moins une substance biologiquement active,

b) de 10 à 35 % en poids d'au moins un sel de sodium, de potassium ou de calcium d'un acide monocarboxylique aliphatique avec 14 à 22 atomes de carbone ou de l'acide ricinoléique,

c) le reste, jusqu'à 100 % en poids, mais au moins 30 % en poids, d'au moins un acide monocarboxylique aliphatique avec 14 à 22 atomes de carbone et/ou d'acide ricinoléique et/ou d'une graisse végétale ou animale durcie.

2. Utilisation du complément alimentaire selon la revendication 1, à une dose journalière de 1 à 100 g pour l'alimentation des ruminants, en particulier des vaches laitières, pour l'amélioration du rendement laitier, de la teneur du lait en protéines et en matières grasses, de la persistance et/ou de la fécondité après le début de la lactation.